# EUROPEAN PATENT SPECIFICATION

(11) **EP 3 852 916 B1**
(45) Date of publication and mention of the grant of the patent: **19.02.2025**
(21) Application number: 19769107.4
(22) Date of filing: 11.09.2019
(51) Int. Cl.: B01J 13/22, A01N 25/28, A61K 8/11, A61K 9/50, C11D 3/50

(54) **IMPROVEMENTS IN OR RELATING TO ORGANIC COMPOUNDS**
VERBESSERUNGEN AN ODER IM ZUSAMMENHANG MIT ORGANISCHEN VERBINDUNGEN
PERFECTIONNEMENTS APPORTÉS AUX COMPOSÉS ORGANIQUES OU EN RELATION AVEC CEUX-CI

(30) Priority: 20.09.2018 GB 201815293
(43) Date of publication of application: 28.07.2021
(73) Proprietor: Givaudan SA, 1214 Vernier (CH)
(72) Inventor: BULGARELLI, Nelly, Loveland, Ohio 45140 (US); HARRISON, Ian, Michael, 78300 Poissy (FR)
(74) Representative: Global Patents
(86) International application number: PCT/EP2019/074154
(87) International publication number: WO 2020/058044

(56) References cited:
- WO-A1-2016/144798
- WO-A1-2016/180769
- WO-A1-2017/004339
- WO-A1-2017/004340
- WO-A1-2017/004343
- US-A1- 2015 099 680

## Description

The present invention relates to a consumer product comprising a plurality of microcapsules dispersed in a dispersing medium, to a method for making a consumer product and to a use of chitosan for enhancing the deposition and rinse resistance of a plurality of microcapsules dispersed in a dispersing medium. A method for enhancing the deposition and rinse resistance of a plurality of microcapsules dispersed in a dispersing medium is also disclosed.

It is known to incorporate encapsulated functional materials in consumer products, such as household care, personal care and fabric care products. Functional materials include for example perfume materials, cosmetic materials, drug materials, and substrate enhancers.

Microcapsules that are particularly suitable for delivery of such functional materials are core-shell microcapsules, wherein the core comprises the functional material and the shell is impervious or partially impervious to the functional material. Usually, these microcapsules are used in aqueous media and the encapsulated functional materials are hydrophobic. A broad selection of shell materials can be used, provided this shell material is impervious or partially impervious to the encapsulated functional material.

Among the functional materials, perfume materials are encapsulated for a variety of reasons. Microcapsules can isolate and protect perfume ingredients from external suspending media, such as consumer product bases, with which they may be incompatible or unstable in. They are also used to assist in the deposition of perfume materials onto substrates, such as skin, hair, fabrics or hard household surfaces. They can also act as a means of controlling the spatiotemporal release of perfume.

Thermosetting resins are common shell materials for such perfume compositions. Core-shell microcapsules formed from aminoplast resins, polyurea resins, polyurethane resins, polyacrylate resins and combinations thereof are generally quite resistant to fragrance leakage when dispersed in aqueous suspending media, even in surfactant-containing media. Furthermore, when incorporated into consumer products, such as laundry detergents or conditioners, they provide perfumery benefits that are unattainable if the perfume is incorporated directly into those products.

In many instances, however, the deposition and adherence of these microcapsules on smooth surfaces and especially on keratinous surfaces, such as skin and hair, are insufficient and the expected benefits associated with the use of microcapsules are not optimal. This is especially the case for rinse-off products involving large amounts of water. In this case, a lack of deposition may be due to the dilution of the microcapsules. Large volumes of rinse water may also wash off the microcapsules from the surface.

WO2016/180769 A1 discloses a benefit agent delivery particle comprising a benefit agent and a chitosan salt at the outer surface of the particle which have good deposition efficiency on tip hair.

WO 2014/064121 A2 discloses benefit agent delivery particles coated with a chitosan salt, which comprises a chitosan component and an organic anion. These particles have been shown to exhibit improved deposition compared to unmodified particles. However, their production requires a rather elaborate pre-formation of a chitosan complex which is then attached to the outer surface of the particles.

It is therefore a problem underlying the present invention to overcome the above-mentioned shortcomings in the prior art. In particular, it is a problem underlying the present invention to provide consumer products comprising microcapsules that show enhanced deposition on keratinous substrates and improved rinse resistance once deposited on these substrates. The microcapsules should be facile to manufacture and versatile with respect to their application. No or only minimal modification of the product composition should be required in connection with their use.

In a first aspect of the present invention, there is provided a consumer product comprising a plurality of microcapsules dispersed in a dispersing medium. The microcapsules comprise a core and a shell around the core. The core comprises at least one functional material. The shells of the microcapsules are coated with chitosan. The dispersing medium comprises additional free chitosan, wherein the chitosan that is coating the shells of the microcapsules and the free chitosan comprised in the dispersing medium have different average molecular weights.

Chitosan is a biopolymer derived from chitin, forming the exoskeleton of crustaceans and preserving the shape of various fungi, such as *Ascomycetes, Zygomycetes, Basidiomycetes* and *Deuteromycetes,* for example *Absidia,* Mucor, *Aspergillus niger, Ganoderma lucidum, Rhizopus oryzae,* and the like. Chitosan production involves the alkaline or enzymatic deacetylation of chitin and is characterized by a deacetylation grade. Both low deacetylation grades, typically below 80 % deacetylation, and high deacetylation grades, typically higher than or equal to 80% deacetylation exist. Deacetylated chitosan is a copolymer consisting of N-acetyl-D-glucosamine an D-glucosamine moieties. The deacetylation grade may be determined by ¹H and/or ¹³C nuclear magnetic resonance spectroscopy. Chitosan is available with molecular weights typically ranging from 3'000 and 5'000'000 g/mol. The molecular weight may be determined by viscosity measurement and/or gel permeation chromatography, according to methods known in the art.

In the context of the present invention, the term *"free chitosan"* is used to describe chitosan which, when the microcapsules are dispersed in a dispersing medium, is separated spatially from the microcapsules by the dispersing medium.

As used herein, a *"consumer product"* means an article intended to be used or consumed in the form in which it is sold, and not intended for subsequent commercial manufacture or modification.

That the shells of the microcapsules are "coated"with chitosan means that the chitosan can be deposited on the shells. On the other hand, the chitosan can also be grafted on or entrapped in the shell of the microcapsules. Grafting and entrapment may be performed by adding the chitosan to the slurry of nascent microcapsules, meaning during the process step where encapsulation place. Alternatively, the chitosan may be grafted on the shell of the formed microcapsule by using coupling agents known to the art.

**In** the context of the present invention, the at least one functional material is particularly selected from the group consisting of a perfume material and a cosmetic material.

In advantageous embodiments of the present invention, the free chitosan comprised in the dispersing medium has a deacetylation grade higher than 60%, more particularly higher than 70%, still more particularly higher than 80%. Chitosans with such high deacetylation grades are more soluble than chitosans having lower acetylation grades and therefore more suitable for the sake of the present invention.

In other advantageous embodiments, the free chitosan comprised in the dispersing medium has a molecular weight between 500'000 and 5'000'000 g/mol, more particularly between 1'000'000and 4'000'000g/mol, still more particularly between 1'500'000and 3'000'000g/mol. Without being bound by any theory, it is reasonable to assume that high molecular weight chitosan adheres better on both keratinous surfaces, in particular hair, and microcapsules.

In other advantageous embodiments of the present invention, the chitosan that is coating the shells of the microcapsules has a deacetylation grade between 60% and 100%, more particularly between 70% and 90%, still more particularly between 75% and 85%. Aqueous solutions of chitosans with such high deacetylation grades are more concentrate and therefore more suitable for coating.

The chitosan that is coating the shells of the microcapsules and the free chitosan comprised in the dispersing medium have different average molecular weights.

The chitosan that is coating the shells of the microcapsules can have a molecular weight between 3'000and 1'000'000g/mol, more particularly between 10'000and 500'000 g/mol, still more particularly between 30'000 and 300'000 g/mol. If the molecular weigt of the chitosan is too low, then the coating may be incomplete, whereas if this molecular weight is too large, then chitosan threads may adsorb on two or more microcapsules and cause microcapsule agglomeration.

The electrical charge present on the microcapsules may be determined by measuring the zeta-potential of these microcapsules. By "*zeta-potential"* (ζ) is meant the apparent electrostatic potential generated by any electrically charged objects in solution, as measured by specific measurement techniques. A detailed discussion of the theoretical basis and practical relevance of the zeta-potential can be found, e.g., in *"*Zeta Potential in Colloid Sciences" (Robert. J. Hunter , Academic Press, London 1981, 1988). The zeta-potential of an object is measured at some distance from the surface of the object and is generally not equal to and lower than the electrostatic potential at the surface itself. Nevertheless, its value provides a suitable measure of the capability of the object to establish electrostatic interactions with other objects present in the solution, such as surfactants, polyelectrolytes and surfaces. The zeta-potential is a relative measurement and its value depends on the way it is measured. In the present case, the zeta-potential of the microcapsules is measured by the so-called phase analysis light scattering method, using a ZetaPALS instrument (ex Brookhaven Instruments Corporation). The zeta-potential of a given object may also depend on the quantity of ions present in the solution. The values of the zeta-potential specified in the present application are measured in an aqeuous buffer solution at desired pH. The ion concentration is 0.001 mol/L.

In advantageous embodiments of the present invention, the coated microcapsules have a zeta potential of:
- Between 0 mV and +50 mV, more particularly between +5 mV and +40 mV, still more particularly between +10 mV and +30 mV, at a pH of 4 and an ion concentration of 0.001 mol/L;
- Between-20 mV and +20 mV, more particularly between-10 mV and +10 mV, still more particularly between-5 mV and +5 mV, at a pH 5.4 and ion concentration of 0.001 mol/L;
- Less than -20 mV, more particularly less than -40 mV, still more particularly less than - 50 mV, preferably between-50 mV and -60 mV, at a pH of 7 and an ion concentration of 0.001 mol/L.

Without being bound by theory, it is assumed that, with such pH dependence of the zeta potential, optimal conditions for both coating at pH below 5 and free chitosan-mediated adhesion on keratinous surface at pH 5 or above are achieved.

In advantageous embodiments of the present invention, the microcapsules have volume-average size from 0.5 to 25 micrometres, more particularly from 1 to 20 micrometres, still more particularly from 5 to 15 micrometres, for example 10 ± 2 micrometres. Microcapsules having such size provide optimal balance between storage stability with respect to leakage of the encapsulated functional material, depostion on hair and mechanical frangibility.

Microcapsule size can be determined in a manner known in the art. A particular method of measuring particle size is light scattering. Light scattering measurements can be made using a Malvern Mastersizer 2000S instrument and the Mie scattering theory. The principle of the Mie theory and how light scattering can be used to measure droplet size and can be found, for example in H. C. van de Hulst, Light scattering by small particles. Dover, New York, 1981. The primary information provided by static light scattering is the angular dependence of the light scattering intensity, which in turn is linked to the size and shape of the droplets. However, in a standard operation method, the size of a sphere having a size equivalent to the size of the diffracting object, whatever the shape of this object, is calculated by the Malvern proprietary software provided with the apparatus. In case of polydisperse samples, the angular dependence of the overall scattering intensity contains information about the size distribution in the sample. The output is a histogram representing the total volume of droplets belonging to a given size class as a function of the capsule size, whereas an arbitrary number of 50 size classes can be chosen. Thus, the size obtained is referred to as volume-average particle size.

Experimentally, a few drops of slurry are added to a circulating stream of degassed water flowing through a scattering cell. The angular distribution of the scattering intensity is measured and analysed by Malvern proprietary software to provide the average size and size-distribution of the droplets present in the sample. In the case of an unimodal (monodisperse) droplet distribution the percentiles Dv(10), Dv(50) and Dv(90) are used as characteristics of the droplets size distribution, whereas Dv(50) corresponds to the median of the distribution and is taken as a measure of the volume-average size of the microcapsules.

In advantageous embodiments, microcapsules, in particular with a volume-average size of 10 ± 2 micrometres, have an chitosan coating to encapsulated functional material ratio is from about 0.015 to about 0.025, more particularly from about 0.018 to about 0.023. Such chitosan coating to encapsulated functional material provides the desired pH-dependence of the zeta-potential described herein above.

**In** particular advantageous embodiments of the invention, the dispersing medium additionally comprises an anionic surfactant.

Typical anionic surfactants include but are not limited to sodium lauryl sulfate, sodium laureth sulfate, sodium trideceth sulfate, ammonium lauryl sulphate, ammonium laureth sulphate, potassium laureth sulfate, sodium tridecyl benzene sulfonate, sodium dodecyl benzene sulfonate, sodium xylene sulfonate, monoethanolamine lauryl sulfate, monoethanolamine laureth sulfate, triethanolamine lauryl sulfate, triethanolamine laureth sulfate, lauryl sarcosine, cocoyl sarcosine, sodium lauryl sarcosinate, sodium lauroyl sarcosinate, triethylamine lauryl sulfate, triethylamine laureth sulfate, diethanolamine lauryl sulfate, diethanolamine laureth sulfate, lauric monoglyceride sodium sulfate, ammonium cocoyl sulfate, ammonium lauroyl sulfate, sodium cocoyl sulfate, sodium lauroyl sulfate, sodium cocoyl isethionate, potassium cocoyl sulfate, potassium lauryl sulfate, monoethanolamine cocoyl sulfate, monoetha nolamine lauryl sulfate, triethanolamine lauryl sulfate, sodium hydroxyethyl-2-decyl ether sulfates, sodium methyl-2-hydroxydecyl ether sulfates, sodium hydroxyethyl-2-dodecyl ether sulfates, sodium monoethoxylated lauryl alkyl sulfates, C₁₂-C₁₈ alkyl sulfonates, ethoxylated or native linear and ramified C₁₂-C₁₈ alcohol sulfates, ethoxylated or native linear and ramified C₁₂-C₁₈ alcohol sulfates, and mixtures thereof.

The role of the anionic surfactant according to the present invention is not only to act as cleaning agent, but also to assist the deposition of free chitosan onto the keratinous substate. Without being bound by theory, anionic surfactants are assumed to form complexes with chitosan which show better adherence on the substrate.

**In** advantageous embodiments of the invention, the anionic surfactant is selected from the group consisting of fatty acid sulphates, laureth sulphates, sarcosines and sarcosinates. The advantages of these particular anionic surfactants lie in theirof cleaning power.

The shell of the microcapsules may comprise shell-forming materials selected from the group consiting of inorganic materials, such as silicate, metals and metal oxides, or organic materials, such as surfactants, natural, semi-synthetic and synthetic organic polymers, and mixtures thereof. Particularly suitable are shells comprising silicates, gelatin, gelatin/gum arabicum complexes, gelatin/carboxymethyl cellulose complexes, alginate/calcium complexes, surfactant lamellar phases, and thermosetting resins, such as aminoplast resins, polyurea resins, polyurethane resins and polyacrylatre resins, and mixtures thereof. Microcapsules having shells comprising such thermosetting resin are particularly resistant to leakage of the uncapsulated functional material over time, while still providing optimal frangibility and release of the functional material when submitted to mechanical stresses.

In other advantageous embodiments of the invention, the consumer product comprises from 0.01to 5 wt.-%, more particularly from 0.1to 2.5 wt.-%, still more particularly from 0.2 to 1 wt.-% of microcapsules, referred to the total weight of the consumer product.

In other advantageous embodiments of the invention, the consumer product comprises from 0.00001 to 0.005 wt.-%, more particularly from 0.00005 to 0.001 wt.-%, still more particularly from 0.00008 to 0.0005 wt.-% of free chitosan, referred to the total weight of the consumer product.

The presence of free chitosan in a product can be recognized by the onset of filament-like structures that are visible under a conventional light microscope, operating under transmitted light and bright field conditions and with a magification of 100 to 200. The typical section of such filament is typically between about 10 micrometres and about 50 micrometres and their length may be as large as 1 mm and more. Without being bound by any theory, the applicant believes that the formation of these filament-like structures is the result of chitosan complexation with anionic surfactants present in the product.

Typical consumer products concerned by the present invention include personal care cleaning and cleansing compositions, such as shampoos, bath and shower gels and liquid soaps.

In other advantageous embodiments of the present invention, the pH of the consumer product is from 4 to 9, more particularly from 5 to 8, and still more particularly from 5.5 to 6.5. Such pH conditions provide optimal stability of the system comprising the dispersing medium, the coated microcapsules and the free chitosan.

Typical formulations of ingredients for use in shampoo with microcapsules according to the present invention may be found, for example, in EP 0 191 564 A2 or WO 1997/023194 A1.

The microcapsules are preferably at a level of 0.01 to 5 wt.-%, more particularly from 0.1 to 2.5 wt.-% and still more particularly from 0.2 to 1 wt.-% of the personal care product, referred to the total weight of the shampoo composition.

In another embodiment of the present invention, the consumer product is a liquid soap comprising one or more anionic surfactants, and other surfactants that may be selected from the group consisting of aminoxide surfactants, non-ionic surfactants, zwitterionic surfactants, and mixtures thereof, mixtures of fatty acids and neutralized fatty acids, electrolytes, one or more preservative, and optionally benefit agents that may be selected from the group consisting of pH-control agents, skin care agents, moisturizers, emollients, thickeners, vitamins, nutrients and dyes.

Typical formulations of ingredients for use in liquid soaps may be found, for example, in CA 2812137 A1 or US 2003/0050200 A1.

In another embodiment of the present invention, the consumer product is a shower gel comprising one or more anionic surfactant, and other surfactantsthat may be selected from the group consisting of mixtures of fatty acids and neutralized fatty acids, aminoxide surfactants, non-ionic surfactants, zwitterionic surfactants, aminoxide surfactants, aminoxide surfactants, and mixtures thereof, electrolytes, one or more preservative, and optionally benefit agents that may be selected from the group consisting of thickeners, pH-control agents skin care agents, moisturizers, emollients, thickeners, vitamins, nutrients, dyes, and the like.

Typical formulations of ingredients for use in shower gels may be found, for example, in US 5,607,678 or US 2012/0263668 A1.

A broad selection of functional materials may be employed in connection with the present invention. The core of the capsules may in particular comprise a hydrophobic material selected from the group consisting of perfume materials, oils, essential oils, fragrance oils, biocides, pheromones, cosmetic materials and topical drugs.

A list of perfume materials that may be encapsulated in accordance with the present invention may be found in the perfumery literature, for example *"Perfume & Flavor Chemicals",* S. Arctander (Allured Publishing, 1994). Encapsulated perfume according to the present invention comprise preferably perfume ingredients selected from ADOXAL^{™} (2,6,10-trimethylundec-9-enal), AGRUMEX^{™} (2-(tert-butyl)cyclohexyl acetate), decanal, 2-methyldecanal, undec-10-enal, undecanal, dodecanal, 2-methylundecanal, (E)-undec-9-enal, (E)-dodec-2-enal), ALLYL AMYL GLYCOLATE (allyl 2-(isopentyloxy)acetate), ALLYL CYCLOHEXYL PROPIONATE (allyl 3-cyclohexylpropanoate, allyl heptanoate, AMBER CORE^{™} (1-((2-(tert-butyl)cyclohexyl)oxy)butan-2-ol), AMBERMAX^{™} (1,3,4,5,6,7-hexahydro-.beta.,1,1,5,5-pentamethyl-2H-2,4a-methanonaphthal-ene-8-ethanol), AMYL SALICYLATE (pentyl 2-hydroxybenzoate), APHERMATE (1-(3,3-dimethylcyclohexyl)ethyl formate), BELAMBRE^{™} ((1R,2S,4R)-2'-isopropyl-1,7,7-trimethylspiro[bicyclo[2.2.1]heptane-2,4'-[1,3]dioxane]), BIGARYL (8-(sec-butyl)-5,6,7,8-tetrahydroquinoline), BOISAMBRENE^{™} FORTE^{™} ((ethoxymethoxy)cyclododecane), BOISIRIS^{™} ((1S,2R,5R)-2-ethoxy-2,6,6-trimethyl-9-methylenebicyclo[3.3.1]nonane), BORNYL ACETATE ((2S,4S)-1,7,7-trimethylbicyclo[2.2.1]heptan-2-yl acetate), BUTYL BUTYRO LACTATE (1-butoxy-1-oxopropan-2-yl butyrate), BUTYL CYCLOHEXYL ACETATE PARA (4-(tert-butyl)cyclohexyl acetate), CARYOPHYLLENE ((Z)-4,11,11-trimethyl-8-methylenebicyclo[7.2.0]undec-4-ene), CASHMERAN^{™} (1,1,2,3,3-pentamethyl-2,3,6,7-tetrahydro-1H-inden-4(5H)-one), CASSYRANE^{™} (5-tert-butyl-2-methyl-5-propyl-2H-furan), CITRAL ((E)-3,7-dimethylocta-2,6-dienal) CITRATHAL^{™} R ((Z)-1,1-diethoxy-3,7-dimethylocta-2,6-diene), CITRONELLAL (3,7-dimethyloct-6-enal), CITRONELLOL (3,7-dimethyloct-6-en-1-ol), CITRONELLYL ACETATE (3,7-dimethyloct-6-en-1-yl acetate), CITRONELLYL FORMATE (3,7-dimethyloct-6-en-1-yl formate), CITRONELLYL NITRILE (3,7-dimethyloct-6-enenitrile), CITRONELLYL PROPIONATE (3,7-dimethyloct-6-en-1-yl propionate), CLONAL (dodecanenitrile), CORANOL (4-cyclohexyl-2-methylbutan-2-ol), COSMONE^{™} ((Z)-3-methylcyclotetradec-5-enone), CYCLAMEN ALDEHYDE (3-(4-isopropylphenyl)-2-methylpropanal), CYCLOGALBANATE (allyl 2-(cyclohexyloxy)acetate), CYCLOHEXYL SALICYLATE (cyclohexyl 2-hydroxybenzoate), CYCLOMYRAL (8,8-dimethyl-1,2,3,4,5,6,7,8-octahydronaphthalene-2-carbaldehyde), DAMASCENONE ((E)-1-(2,6,6-trimethylcyclohexa-1,3-dien-1-yl)but-2-en-1-one), DAMASCONE ALPHA ((E)-1-(2,6,6-trimethylcyclohex-2-en-1-yl)but-2-en-1-one), DAMASCONE DELTA ((E)-1-(2,6,6-trimethylcyclohex-3-en-1-yl)but-2-en-1-one), (E)-dec-4-enal), DELPHONE (2-pentylcyclopentanone), DIHYDRO ANETHOLE (1-methoxy-4-propylbenzene), DIHYDRO JASMONE (3-methyl-2-pentylcyclopent-2-enone), DIMETHYL BENZYL CARBINOL (2-methyl-1-phenylpropan-2-ol), DIMETHYL BENZYL CARBINYLACETATE (2-methyl-1-phenylpropan-2-yl acetate), DIMETHYL BENZYL CARBINYLBUTYRATE (2-methyl-1-phenylpropan-2-yl butyrate), 4,7-dimethyloct-6-en-3-one, DIMETOL (2,6-dimethylheptan-2-ol), DIPENTENE (1-methyl-4-(prop-1-en-2-yl)cyclohex-1-ene), DUPICAL^{™} ((E)-4-((3aS,7aS)-hexahydro-1H-4,7-methanoinden-5(6H)-ylidene)butanal), EBANOL^{™} ((E)-3-methyl-5-(2,2,3-trimethylcyclopent-3-en-1-yl)pent-4-en-2-ol), ethyl hexanoate, ethyl octanoate, ETHYL LINALOOL ((E)-3,7-dimethylnona-1,6-dien-3-ol), ETHYL LINALYL ACETATE ((Z)-3,7-dimethylnona-1,6-dien-3-yl acetate), ethyl heptanoate, ETHYL SAFRANATE (ethyl 2,6,6-trimethylcyclohexa-1,3-diene-1-carboxylate), EUCALYPTOL ((1s,4s)-1,3,3-trimethyl-2-oxabicyclo[2.2.2]octane), FENCHYL ACETATE ((2S)-1,3,3-trimethylbicyclo[2.2.1]heptan-2-yl acetate), FENCHYL ALCOHOL ((1S,2R,4R)-1,3,3-trimethylbicyclo[2.2.1]heptan-2-ol), FlXOLIDE^{™} (1-(3,5,5,6,8,8-hexamethyl-5,6,7,8-tetrahydronaphthalen-2-yl)ethanone), FLORALOZONE^{™} (3-(4-ethylphenyl)-2,2-dimethylpropanal), FLORHYDRAL (3-(3-isopropylphenyl)butanal), FLOROCYCLENE^{™} ((3aR,6S,7aS)-3a,4,5,6,7,7a-hexahydro-1H-4,7-methanoinden-6-yl propionate), FLOROPAL^{™} (2,4,6-trimethyl-4-phenyl-1,3-dioxane), FRESKOMENTHE^{™} (2-(sec-butyl)cyclohexanone), FRUITATE ((3aS,4S,7R,7aS)-ethyl octahydro-1H-4,7-methanoindene-3a-carboxylate), FRUTONILE (2-methyldecanenitrile), GALBANONE^{™} PURE (1-(3,3-dimethylcyclohex-1-en-1-yl)pent-4-en-1-one), GARDOCYCLENE^{™} ((3aR,6S,7aS)-3a,4,5,6,7, 7a-hexahydro-1H-4,7-methanoinden-6-yl isobutyrate), GERANIOL ((E)-3,7-dimethylocta-2,6-dien-1-ol), GERANYL ACETATE SYNTHETIC ((E)-3,7-dimethylocta-2,6-dien-1-yl acetate), GERANYL ISOBUTYRATE ((E)-3, 7-dimethylocta-2,6-dien-1-yl isobutyrate), GIVESCONE^{™} (ethyl 2-ethyl-6,6-dimethylcyclohex-2-enecarboxylate), HABANOLIDE^{™} ((E)-oxacyclohexadec-12-en-2-one), HEDIONE^{™} (methyl 3-oxo-2-pentylcyclopentaneacetate), HERBANATE^{™} ((2S)-ethyl 3-isopropylbicyclo[2.2.1]hept-5-ene-2-carboxylate), HEXENYL-3-CIS BUTYRATE ((Z)-hex-3-en-1-yl butyrate), HEXYL CINNAMIC ALDEHYDE ((E)-2-benzylideneoctanal), HEXYL ISOBUTYRATE (hexyl isobutyrate), HEXYL SALICYLATE (hexyl 2-hydroxybenzoate), INDOFLOR^{™} (4,4a,5,9b-tetrahydroindeno[1,2-d][1,3]dioxine), IONONE BETA ((E)-4-(2,6,6-trimethylcyclohex-1-en-1-yl)but-3-en-2-one), IRISONE ALPHA ((E)-4-(2,6,6-trimethylcyclohex-2-en-1-yl)but-3-en-2-one), IRONE ALPHA ((E)-4-(2,5,6,6-tetramethylcyclohex-2-en-1-yl)but-3-en-2-one), ISO E SUPER^{™} (1-(2,3,8,8-tetramethyl-1,2,3,4,5,6,7,8-octahydronaphthalen-2-yl)ethanone), ISOCYCLOCITRAL(2,4,6-trimethylcyclohex-3-enecarbaldehyde), ISONONYLACETATE (3,5,5-trimethylhexyl acetate), ISOPROPYL METHYL-2-BUTYRATE (isopropyl 2-methyl butanoate), ISORALDEINE^{™} 70 ((E)-3-methyl-4-(2,6,6-trimethylcyclohex-2-en-1-yl)but-3-en-2-one), JASMACYCLENE^{™} ((3aR,6S,7aS)-3a,4,5,6,7,7a-hexahydro-1H-4,7-methanoinden-6-yl acetate), JASMONE CIS ((Z)-3-methyl-2-(pent-2-en-1-yl)cyclopent-2-enone), KARANAL^{™} (5-(sec-butyl)-2-(2,4-dimethylcyclohex-3-en-1-yl)-5-methyl-1,3-dioxane), KOAVONE ((Z)-3,4,5,6,6-pentamethylhept-3-en-2-one), LEAF ACETAL ((Z)-1-(1-ethoxyethoxy)hex-3-ene), LEMONILE^{™} ((2E,6Z)-3,7-dimethylnona-2,6-dienenitrile), LIFFAROME^{™} GIV ((Z)-hex-3-en-1-yl methyl carbonate), LILIAL^{™} (3-(4-(tert-butyl)phenyl)-2-methylpropanal), LINALOOL (3,7-dimethylocta-1,6-dien-3-ol), LINALYL ACETATE (3,7-dimethylocta-1,6-dien-3-yl acetate), MAHONIAL^{™} ((4E)-9-hydroxy-5,9-dimethyl-4-decenal), MALTYL ISOBUTYRATE (2-methyl-4-oxo-4H-pyran-3-yl isobutyrate), MANZANATE (ethyl 2-methylpentanoate), MELONAL^{™} (2,6-dimethylhept-5-enal), MENTHOL (2-isopropyl-5-methylcyclohexanol), MENTHONE (2-isopropyl-5-methylcyclohexanone), METHYL CEDRYL KETONE (1-((1S,8aS)-1,4,4,6-tetramethyl-2,3,3a,4,5,8-hexahydro-1H-5,8a-methanoazulen-7-yl)ethanone), METHYL NONYL KETONE EXTRA (undecan-2-one), METHYL OCTYNE CARBONATE (methyl non-2-ynoate), METHYL PAMPLEMOUSSE (6,6-dimethoxy-2,5,5-trimethylhex-2-ene), MYRALDENE (4-(4-methylpent-3-en-1-yl)cyclohex-3-enecarbaldehyde), NECTARYL (2-(2-(4-methylcyclohex-3-en-1-yl)propyl)cyclopentanone), NEOBERGAMATE^{™} FORTE (2-methyl-6-methyleneoct-7-en-2-yl acetate), NEOFOLIONE^{™} ((E)-methyl non-2-enoate), NEROLIDYLE^{™} ((Z)-3,7,11-trimethyldodeca-1,6,10-trien-3-yl acetate), NERYLACETATE HC((Z)-3,7-dimethylocta-2,6-dien-1-yl acetate), NONADYL (6,8-dimethylnonan-2-ol), NONENAL-6-CIS ((Z)-non-6-enal), NYMPHEAL^{T}"' (3-(4-isobutyl-2-methylphenyl)propanal), ORIVONE^{™} (4-(tert-pentyl)cyclohexanone), PARADISAMIDE^{™} (2-ethyl-N-methyl-N-(m-tolyl)butanamide), PELARGENE (2-methyl-4-methylene-6-phenyltetrahydro-2H-pyran), PEONILE^{™} (2-cyclohexylidene-2-phenylacetonitrile), PETALIA^{™} (2-cyclohexylidene-2-(o-tolyl)acetonitrile), PIVAROSE^{™} (2,2-dimethyl-2-pheylethyl propanoate), PRECYCLEMONE^{™} B (1-methyl-4-(4-methylpent-3-en-1-yl)cyclohex-3-enecarbaldehyde), PYRALONE^{™} (6-(sec-butyl)quinoline), RADJANOL^{™} SUPER ((E)-2-ethyl-4-(2,2,3-trimethylcyclopent-3-en-1-yl)but-2-en-1-ol), RASPBERRY KETONE (N112) (4-(4-hydroxyphenyl)butan-2-one), RHUBAFURANE^{™} (2,2,5-trimethyl-5-pentylcyclopentanone), ROSACETOL (2,2,2-trichloro-1-phenylethyl acetate), ROSALVA (dec-9-en-1-ol), ROSYFOLIA ((1-methyl-2-(5-methylhex-4-en-2-yl)cyclopropyl)-methanol), ROSYRANE^{™} SUPER (4-methylene-2-phenyltetrahydro-2H-pyran), SERENOLIDE (2-(1-(3,3-dimethylcyclohexyl)ethoxy)-2-methylpropyl cyclopropanecarboxylate), SILVIAL^{™} (3-(4-isobutylphenyl)-2-methylpropanal), SPIROGALBANONE^{™} (1-(spiro[4.5]dec-6-en-7-yl)pent-4-en-1-one), STEMONE^{™} ((E)-5-methylheptan-3-one oxime), SUPER MUGUET^{™} ((E)-6-ethyl-3-methyloct-6-en-1-ol), SYLKOLIDE^{™} ((E)-2-((3,5-dimethylhex-3-en-2-yl)oxy)-2-methylpropyl cyclopropanecarboxylate), TERPINENE GAMMA (1-methyl-4-propan-2-ylcyclohexa-1,4-diene), TERPINOLENE (1-methyl-4-(propan-2-ylidene)cyclohex-1-ene), TERPINYL ACETATE (2-(4-methylcyclohex-3-en-1-yl)propan-2-yl acetate), TETRAHYDRO LINALOOL (3,7-dimethyloctan-3-ol), TETRAHYDRO MYRCENOL (2,6-dimethyloctan-2-ol), THIBETOLIDE (oxacyclohexadecan-2-one), TRIDECENE-2-NITRILE ((E)-tridec-2-enenitrile), UNDECAVERTOL ((E)-4-methyldec-3-en-5-ol), VELOUTONE^{™} (2,2,5-trimethyl-5-pentylcyclopentanone), VIRIDINE^{™} ((2,2-dimethoxyethyl)benzene), ZINARINE^{™} (2-(2,4-dimethylcyclohexyl)pyridine), and mixtures thereof.

The perfume materials and cosmetic materials to be encapsulated in the encapsulated compositions are preferably hydrophobic. Preferably, the cosmetic materials have a calculated octanol/water partition coefficient (ClogP) of 1.5 or more, more preferably 3 or more. Preferably, the ClogP of the cosmetic materials is from 2 to 7.

Particularly useful cosmetic materials may be selected from the group consisting of emollients, smoothening actives, hydrating actives, soothing and relaxing actives, decorative actives, deodorants, anti-aging actives, draining actives, remodelling actives, skin levelling actives, preservatives, anti-oxidant actives, antibacterial or bacteriostatic actives, cleansing actives, lubricating actives, structuring actives, hair conditioning actives, whitening actives, texturing actives, softening actives, anti-dandruff actives, and exfoliating actives.

Particularly useful cosmetic materials include, but are not limited to, hydrophobic polymers, such as alkyldimethylsiloxanes, polymethylsilsesquioxanes, polyethylene, polyisobutylene, styrene-ethylene-styrene and styrene-butylene-styrene block copolymers, and the like, mineral oils, such as hydrogenated isoparaffins, silicone oils and the like, vegetable oils, such as argan oil, jojoba oil, aloe vera oil, and the like , fatty acids and fattyalcohols and their esters, glycolipide , phospholipides, sphingolipides, such as ceramides , sterols and steroids, terpenes, sesquiterpenes, triterpenes and their derivatives, essential oils, such as arnica oil, artemisia oil, bark tree oil, birch leaf oil, calendula oil, cinnamon oil, echinacea oil, eucalyptus oil, ginseng oil, jujube oil, helianthus oil, jasmine oil, lavender oil, lotus seed oil, perilla oil, rosmary oil, sandal wood oil, tea tree oil, thyme oil, valerian oil, wormwood oil, ylang ylang oil, yucca oil and the like.

**In** an embodiment of the present invention, the cosmetic material may be selected from the group consisting of sandal wood oil, such as fusanus spicatus kernel oil, panthenyl triacetate (CAS-No.: 94089-18-6), tocopheryl acetate, tocopherol, naringinin (CAS-No.: 480-41-1), ethyl linoleate, farnesyl acetate, farnesol, citronellyl methyl crotonate (CAS-No.: 20770-40-5), ceramide-2 (1-stearoiyl-C18-sphingosine, CAS-No: 100403-19-8), and mixtures thereof.

The functional material may optionally be admixed with various hydrophobic excipients, such as apolar solvents, oils, waxes and apolar polymers.

Consumer products according to the present invention show improved micro-capsule deposition and rinse resistance on keratinous substrates, compared consumer products comprising polymer-coated microcapsules known to the art.

Once deposited on the keratinous surfaces, the microcapsules are able to release their functional material by diffusion through the microcapsule shell or following the mechanical rupture of the microcapsule shell. Mechanical rupture may follow a mechanical action, such as rubbing, squeezing, combing, washing and the like or heating, for example using a hair dryer.

Diffusion-mediated release is particularly desired if the functional material is a fragrance composition, because, in this case, a nice smell may be perceived over a long time, for example several hours, after application of the microcapsules on the substrate. On the other hand, a mechanical rupture may provoke a surprising and pleasant boost of odor.

In a second aspect of the present invention, there is provided a method for making a consumer product, in particular a consumer product as described herein above. The method comprises the steps of:
- Providing a plurality of microcapsules, the microcapsules comprising a core and a shell around the core, the core comprising at least one functional material;
- Providing a dispersing medium;
- Dispersing the microcapsules in the dispersing medium;
- Coating the plurality of core-shell microcapsules with chitosan to obtain a plurality of coated core-shell microcapsules;
- Adding additional free chitosan to the dispersing medium, wherein the chitosan that is coating the shells of the microcapsules and the free chitosan comprised in the dispersing medium have different average molecular weights.

In particularly advantageous embodiments of the invention, the consumer products may be formed according to a method comprising, preferably consisting of, the following steps:
a) Adjusting the pH of a microcapsule slurry, having a solid content between 8 and 50 wt%, more particularly between 20 and 47 wt%, still more particularly between 35 and 45 wt% of uncoated microcapsules, to a value of from 2.5 to 4.5, preferably 3.5 ± 0.2;
b) Adding slowly a first chitosan C1 in powder form to the microcapsule slurry under stirring at a rate of addition from 0.001 kg chitosan C1 per kg of uncoated microcapsule per hour to 0.1 kg chitosan C1 per kg of uncoated microcapsule per hour until full coating of said uncoated microcapsules is achieved, wherein the level of chitosan C1 for full coating is determined as the level at which a slurry of coated microcapsule colours an aqueous ninhydrin solution. The optimal rate of addition may change if the slurry is diluted, or depending on the geometry of the vessel and of the stirrer. A crucial aspect of the process parameter optimization at this stage is to avoid aggregation of the microcapsules;
c) Adjusting the pH to a value of from 4.5 to5.5, preferably 5 ± 0.2;
d) Keeping the mixture obtained in c) under stirring for several hours, for example 6 hours, 8 hours, 12 hours or more, at a temperature lower than 60 °C, for example at room temperature, in order to obtain a slurry of chitosan-coated microcapsules;
e) Preparing a second 2 wt% chitosan solution by solubilizing second chitosan C2 in an aqueous solution of acetic acid or citric acid having a pH from 3 to 4, for example 3.5, under vigorous stirring using a propeller stirrer;
f) Adding the second chitosan C2 solution to the consumer product in order to obtain a level from 0.00001 to 0.005 wt%, more particularly from 0.00005 to 0.001 wt%, still more particularly from 0.00008 to 0.0005 wt% of free chitosan, referred to the total weight of the consumer product;
g) Adding the slurry of chitosan-coated microcapsules to the consumer product and dispersing these microcapsules therein under stirring, so that the level coated microcapsule in the consumer product is from 0.01 to 5 wt%, more particularly from 0.1 to 2.5 wt% and still more particularly from 0.2 to 1 wt% of coated microcapsules in the product. This step may be optionally preceded by a dilution step, where the slurry is diluted from 2 to 25, more particularly from 5 to 20, for example 10 times with deionized water.

With regard to step b), the extent of coating may be expressed as the ratio of polymer weight added to the slurry to the weight of the encapulated functional material present in the slurry. The optimal chitosan coating to encapsulated functional material ratio may depend not only of the size and size distribution of the microcapsules, but also on the number of anionic groups present on the surface of the microcapsules and on the deacetylation grade of the chitosan, among other parameters. Hence, the optimal coating to microcapsules ratio may be different from case to case. For example, the optimal coating to encapsulated functional material ratio may vary from 0.005 to 0.5 and more, and have very diverse values such as 0.07, 0.012, 0.03, 0.05, 0.15, and 0.25. The optimal coating to encapsulated functional material ratio is the point where substantially all cationic groups present on the chitosan molecules present in the slurry are bound to the microcapsules. This point coincides with the onset of a dark blue colour in the so-called ninhydrin colorimetric test. This test is based on the property of primary amines to react with ninhydrin (2,2-Dihydroxy-1H-indene-1,3(2H)-dione , CAS No 485-47-2) to form a dye known under the name of Ruhemann Violet (3-Hydroxy-2-[(1,3-dioxo-indanyliden-(2))-amino]-indenon-(1), CAS No 7185-16-2), according to a chemical mechanism known to the art (see for example C. B. Bottom et al. Biochemical Education 6 (2010) 4-5). The way the ninhydrine test is performed according to the present invention is described in Example 2 hereinunder.

Although adding to the slurry one more of a suspending agent, a preservative or any other conventional excipients is optional, it is conventional to stabilize a slurry by adding certain well-known excipients.

For example, once the slurry is formed, one can add a suspending agent to it in order to prevent the slurry from phase-separating, such as creaming or sedimenting. Any of the conventional suspending agents may be employed in the present invention. Coventional suspending agents include but are not limited to a hydrocolloid selected from the group consisting of starch and starch derivatives, such as modified starch, dextrin, maltodextrin, gums, such as gum arabic or gum accacia, xanthan gum, gum tragacanth, gum karaya, guar gum, cellulose and cellulose derivatives, such as carboxy methyl cellulose, hydroxyethyl cellulose, hydroxyethyl cellulose/lauryl-dimethylammoniumepoxy condensat, hydroxypopyl cellulose, cationic cellulose (for example polyquaternium-4), cellulose gum, carrageenan, agar-agar, pectines and pectic acid, gelatine, protein hydrolysates, polymer and copolymers of vinyl and allyl monomers, such as polyvinylpyrrolidone, poly(vinyl pyrrolidone-co-vinylacetate), poly(vinyl alcohol-co-vinyl acetate) (more particularly hydrolyzed polyvinylacetates having a degree of hydrolysis between 85 and 92%), vinyl ester homopolymers and copolymers, such as vinyl acetate, vinyl pivalate, vinyl versatate, poly(vinyl methyl ether), poly(vinyl alkyl amines), such as poylvinylmethylamine, quaternized polyvinyl alkyl amines, vinyl pyridine and quaternized vinyl pyridine, vinyl imidazoline, vinyl imidazole, vinyl imidazolinium, dimethyldiallyl ammonium chloride, vinyl sulphonate homopolymers and/or copolymers, polyamines and polyimines, ethoxylated polyamines, polymers, copolymers and cross-polymers derived from (meth)acryloyl monomers, such as methyl methyl acrylate, ethyl methacrylate, 2-ethyl-hexyl acrylate, lauryl methacrylate, C₁₀-C₃₀ alkyl acrylate, hydroxyalkyl (meth)acrylate, such as 2-hydroxypropyl acrylate and 2-hydroxypropyl methacrylate, acrylamidodimethyl taurate, aryl (meth)acrylates, such as phenyl acrylate and benzyl acrylate, (meth)acrylic acids and their salts, such as sodium and potassium (meth)acrylates, sodium acryloyldimethyltaurate, (meth)acrylamides, N-alkyl (meth)acrylamides, such as N,N-dimethylaminoalkyl methacrylate, quaternized N-alkyl (meth)acrylamides, such as methacrylamidopropyl-trimethylammonium chloride, acrylamidoe-thyltrimonium chloride, acrylamidolauryltrimethylammonium chloride, and (meth)acrylamido alkyl sulphonates poly(maleic anhydride) and poly(maleic anhydride-co-vinyl ether), and their hydrolysates, poly(acrylic acid-co-maleic acid)copolymer, poly(alkyleneoxide), polyurethanes and polyureas, such as anionic, cationic non-ionic and amphoteric polyurethanes and polyureas, mixed copolymers thereof, and mixture thereof.

**It** isalso conventional to add biological preservatives to aqueous slurries to prevent unwanted growth of moulds and other microorganisms.

Suitable preservatives include, but are no limited to quaternary compounds, biguanide compounds (CAS No.: 32289-58-0, 27083-27-8, 28757-47-3, 133029-32-0), poylaminopropyl biguanidine, hexetidine, para-chloro-meta-cresol, methenamine, 3-bis(hydroxymethyl)-5,5-dimethylimidazolidine-2,4-dione, quaternium-15, benzoic acid, salicylic acid, undec-10-enoic acid, formic acid, biphenyl-2-ol and their salts, 4-hydroxybenzoic acid and its esters and salts, sorbic acid and its salts, isothiazolinones, 2-bromo-2-nitro-1,3-propanediol, 5-bromo-5-nitro-1,3-dioxane, 2-(thiazol-4-yl) benzimidazole, benzimidazole carbamate, 3-(4-ohlorophenyl)-1-(3,4-dichlorophenyl)uree, 3-iodo-2-propynylbutylcarbamate, ethyl(2-mercaptobenzoato-(2-)-O,S) mercurate(1-) sodium, 5-chloro-2-(2,4-dichlorophénoxy)phenol, dichlorobenzyl alcohol, chloroxylenol, imidazolidinyl urea, phenoxyethanol, benzyl alcohol and mixtures thereof.

In advantageous embodiments of the invention, chitosan is used as biological preservative and the slurry is substantially free of any additional biological preservative.

The slurry may also contain commonly employed adjuvants. The term *"adjuvants"* refers to ingredients that may affect the performance of a composition, other than its hedonic performance. For example, an adjuvant may be an ingredient that acts as an aid to processing a perfume composition or consumer product containing said composition, or it may improve handling or storage of a perfume composition or consumer product. It might also be an ingredient that provides additional benefits such as imparting colour or texture. It might also be an ingredient that imparts light resistance or chemical stability to one or more ingredients contained in a perfume composition or consumer product. A detailed description of the nature and type of adjuvants commonly used in perfume compositions or consumer products cannot be exhaustive, but such ingredients are well known to a person skilled in the art. Examples of adjuvants include solvents, waxes, oils, pigments, dyestuffs and colouring matters, extenders, fillers and reinforcing agents, stabilizers against the detrimental effects of heat and light, bulking agents, acidulants, buffering agents and antioxidants.

Compositions in the form of a slurry of core-shell microcapsules can be incorporated into all manner of consumer products. However, for some applications it might be desirable to add the core-shell microcapsules in the form of a dry powder. Thus, in accordance with the present invention, in an optional step for dehydrating the slurry to form a composition of core-shell microcapsules in powder form, the slurry may be dehydrated to provide the composition of the present invention in dry powder form.

If desired, the microcapsules can be isolated in the form of a dry powder. For example, the solid capsules can be isolated by filtration and dryed. Drying of the isolated capsules may be performed by heating, e.g. in an oven or by contact with a heated gas stream. Preferably, drying of the dispersion is carried out by spray drying or fluid-bed drying. Spray drying techniques and apparatus are well known in the art. A spray-drying process pushes suspended capsules through a nozzle and into a drying chamber. The capsules may be entrained in a fluid (such as air) that moves inside of a drying chamber. The fluid (which may be heated, for example at a temperature of 150 and 120 °C, more preferably between 170 °Cand 200°C, and still more preferably between 175 °C and 185 °C) causes the liquid to evaporate, leaving behind the dried capsules which can then be collected from the process equipment and further processed.

Prior to or after the spray drying step, it may be desirable to add a flow aid, such as silica or the like to the slurry to ensure the realization of fine, free-flowing powdered microcapsules with low surface perfume oil. Flow aids include silicas or silicates, such as precipitated, fumed or colloidal silicas, starches, calcium carbonate, sodium sulphate, modified cellulose, zeolites or other inorganic particulates known in the art.

The slurry of microcapsules may be spray-dried in a conventional spray drying tower, using a two-fluid nozzle or spin-dried in a conventional spin dryer. If desired, at least one hydrocolloid may be added to the microcapsule slurry, as such or in the form of an aqueous solution. Typical hydrocolloids include starch, modified starch such as dextrin-modified with octenyl succinate anhydride, and gum arabic. Optionally, maltodextrins and sugar alcohols, such as sorbitol, mannitol or maltitol may also be added. The hydrocolloid may itself contain a functional material. This functional material may be the same as, or different form, that the functional material present in the core of the capsule. This is achieved by performing the step of (1) emulsifying a second functional material in aqueous hydrocolloid solution, optionally comprising maltodextrins and sugars or sugar alcohols to form a second slurry (2) mixing the second slurry with a slurry of microcapsules comprising a first functional material and (3) drying this mixture. Such a process is described in WO 2007/137441 A1, Example 5.

By means of the present invention it is possible to obtain compositions in the form of a slurry of core-shell microcapsules that have a high core content, typically within the range of 30 to 50 wt %, and more particularly from 35 to 45 wt %.

A method for enhancing the deposition and rinse resistance of a plurality of microcapsules dispersed in a dispersing medium, in particular contained in a consumer product, on a surface, in particular on a keratinous surface is also disclosed. The microcapsules comprise a core and a shell around the core. The core comprise at least one functional material.

The method comprises the steps of:
- Coating the plurality of core-shell microcapsules with chitosan to obtain a plurality of coated core-shell microcapsules;
- Adding additional free chitosan to the dispersing medium.

In another aspect of the invention, there is provided a use of chitosan for enhancing the deposition and rinse resistance of a plurality of microcapsules on a surface, in particular on a keratinous surface. The microcapsules comprise a core and a shell around the core. The core comprises at least one functional material. The microcapsules are dispersed in a dispersing medium, in particular contained in a consumer product. The use involves coating the plurality of microcapsules with chitosan toobtain a plurality of coated microcapsules and adding additional free chitosan to the dispersing medium. The use can be part of an above-described method.

Further advantages and particular features of the present invention become apparent from the following discussion of several examples.

### EXAMPLES

### Example 1: Microcapsule coating with chitosan C1 (not according to the invention)

Aminoplast microcapsules encapsulating a core comprising a fragrance material as functional material were prepared according to the method disclosed as process P5.1 in EP 2 111214 B1. The volume average diameter of the microcapsules, as measured by light scattering, was 10 ± 0.5 micrometres. The solid content of the slurry was 42 wt%, which corresponds to 35.5 wt% encapsulated perfume.

These microcapsules were coated with chitosan C1 by performing the steps of:
a) Adjusting the pH of microcapsule slurry to a value of 3.5 ± 0.2;
b) Adding slowly a known amount of chitosan C1, as reported in Table 1, in powder form to 1000 g of the microcapsule slurry under stirring and over a period of time of 15 minutes and adjusting the pH to a value of 5 ± 0.2;
c) Maintaining the resulting mixture for 6 hours at room temperature, in order to obtain a slurry of chitosan-coated microcapsules.

This procedure was repeated with different a mounts of chitosan C1, so that the ratio chitosan C1 content to functional material content of the slurry (referred to as chitosan to functional material ratio hereinafter) was varied from 0.007 to 0.13 [samples 1.1 to 1.10]. This ratio is used here for the sake of simplicity, considering that the functional material is almost quantitatively encapsulated and therefore varies linearly with the weight of the formed microcapsules.

**Table 1: Initial chitosan to encapsulated functional material ratio**

| Sample | 1.1 | 1.2 | 1.3 | 1.4 | 1.5 | 1.6 | 1.7 | 1.8 | 1.9 | 1.10 |
|---|---|---|---|---|---|---|---|---|---|---|
| Initial chitosan to functional material ratio | 0.007 | 0.01 | 0.014 | 0.021 | 0.028 | 0.042 | 0.056 | 0.07 | 0.098 | 0.133 |
| Supernatant coloration (example3) | None | None | None | Bluish | Blue | Blue | Blue | Blue | Blue | Blue |

### Example 2: Determination of the optimal chitosan coating (not according to the invention)

Samples 1.1 to 1.10 were ultracentrifuged and the pH of their supernatant increased to 6.5 by adding NaOH. Then, 5 ml of a 2 wt% of ninhydrin in deionized water were admixed with 5 ml of supernatant. The development of a velvet color in the mixture is the sign of the presence of free unprotonated amine functional groups and therefore of the presence of free chitosan in the supernatant. On the other hand, the presence of free chitosan in the supernatant is a sign that the surface of the microcapsules is saturated by adsorbed chitosan and fully coated. The onset of the ninhydrin-amine complex can be taken as the point where the optimal chitosan to functional material ratio for quantitative coating has been exceeded. The optimal chitosan to functional material ratio is almost independent of the source, molecular weight and deacetylation grade of chitosan C1 (Table 1). Oppositely, the zeta potential at pH 4 depends on these parameters (see Table 2). It is also observed that the value of the zeta potential reaches a plateau once the quantitative coating has been reached, which confirms that the surface of the capsules is effectively saturated with chitosan C1. The value of the zeta potential at plateau is given in Table 2 for the different types of chitosan.

**Table 2: Optimal chitosan C1 to perfume ratio and zeta potential at the onset of complete coating (microcapsule diameter: 10 µm)**

| Origin of chitosan C1 | Molecularweight of chitosanC1 (averageing/mol, as given by the supplier) | Deacetylation grade of chitosan C1 (as given by the supplier) | Optimal chitosan C1 to perfume ratio for quantitative coating | Zeta potential of coated microcapsules at pH4 in mV |
|---|---|---|---|---|
| Shrimp | 30'000 | 90% | 0.02 | +10 ± 5 mV |
| Shrimp | 200'000 | 75-85% | 0.02 | +25 ± 5 mV |
| Shrimp | 1'700'000 | 90% | 0.02 | +10 ± 5 mV |
| Mushroom | 200'000 | 80 ± 10 %(*) | 0.03 | +20 ± 5 mV |

| | | | | |
|---|---|---|---|---|
| (*) estimate | | | | |

### Example 3: Preparation of shampoo compositions and deposition data (3.1-3.8 not according to the invention)

In a first set of experiments, chitosan C1-coated microcapsule slurries were added to a shampoo composition under gentle stirring with a paddle mixer (example 3.3 and 3.4 in table 3).

In a second set of experiments, 0.005 g of a 2% chitosan C2 solution in deionized water at pH 3.5 were incorporated into 99.995 g of shampoo base under gentle stirring with a propeller and dispersed therein under stirring. Then chitosan C1-coated microcapsule slurries were added under stirring, so that the level of slurry in the shampoo base was 0.5 wt% referred to the total weight of the shampoo base. The characteristics of both chitosan C2 and chitosan C1-coated microcapsule were varied. The mixture was let to macerate overnight before performing the deposition measurements.

In a fourth set of experiments, comparative samples were prepared with uncoated microcapsules and cationic guars instead of chitosan C2.

The shampoo was applied on swatches that have been previously wetted with tap water at a temperature of 37 °C. The amount of shampoo was 10% of the weight of the swatches. The shampoo application was performed by gently massaging the swatches during 20 seconds, waiting for 1 minute, rinsing the swatches with tap water at a temperature of 37 °Cand removing the excess water by sliding each swatch between two fingers vertically from top to bottom. The extent of deposition was determined by image analysis micrographs obtained with a fluorescence light microscope at a magnification of 40 x, using Stream Motion software and Hostasol Yellow 3G as fluorescent agent at 0.02 wt% in perfume, 450 nm excitation wavelength and 500 nm emission wavelength. The deposition value and shampoo composition details are reported in Table 3.

In Table 3, the deposition scores obtained by a combination of chitosan-coated microcapsules and free chitosan, using chitosan with different molecular weights and origins, are compared to the scores obtained with un-coated microcapsules, with cationic microcapsules, with different free cationic polymer (cationic guar) and in the absence of of free chitosan.

**Table 3: Shampoo formulations and deposition data**

| Sample | Nature of polymer coating | Nature of free polymer | Level of free polymer C2 in shampoo | Deposition # capsules / mm² |
|---|---|---|---|---|
| 3.1 | No coating | - | - | 0.3 ± 0.2 |
| 3.2 | No coating | Shrimp chitosan 1'700'000 g/mol | 0.0001 wt% | 0.8 ± 0.4 |
| 3.3 | Cationicaminoplast according to WO 2016207180A1 | - | - | **1.1** ± 0.6 |
| 3.4 | Shrimp chitosan 30'000 g/mol | - | - | 1.9 ± 1 |
| 3.5 | Shrimp chitosan 200'000g/mol | - | - | 2.5 ± 1 |
| 3.6 | Shrimp chitosan 1'700'000 g/mol | - | - | 1.2 ± 0.4 |
| 3.7 | Shrimp chitosan 200'000g/mol | Cationicguar > 1'000'000 g/mol | 0.0001 wt% | 1.1 ± 0.5 |
| 3.8 | Shrimp chitosan 200'000g/mol | Shrimp chitosan 200'000g/mol | 0.0001 wt% | 2.5 ± 1 |
| 3.9 | Shrimp chitosan 200'000g/mol | Shrimp chitosan 1'700'000 g/mol | 0.0001 wt% | 4.6 ± 1 |
| 3.11 | Mushroom chitosan 200'000g/mol | Shrimp chitosan 1'700'000 g/mol | 0.0001 wt% | 4.6 ± 1 |

**Table 4: Model shampoo base composition**

| Ingredient trade name | INCI name | Percentage by weight in shampoo |
|---|---|---|
| PROPYLENE GLYCOL | Propylene Glycol | 1.00 |
| JAGUAR C-13S (ex RHODIA) | Guar Hydroxypropyltrimonium Chloride | 0.25 |
| MARLI NAT 242/28 (ex SASOL) | Sodium Laureth Sulfate | 25.00 |
| DEHYTON AB 30 (ex COGNIS) | Coco Betaine | 5.00 |
| EUPERLAN PK 3000 (ex COGNIS) | Glycol distea rate, La ureth-4 and Cocoamidopropyl Betaine | 0.50 |
| GLYDANT PLUS LIQ (ex LONZA) | DMDM Hydantoin | 0.50 |
| SODIUM CHLORIDE | Sodium Chloride | 1.20 |
| BC 2102 (ex BALLU CHIMIE) | Dimethiconol Emulsion | 2.00 |
| DEIONIZED WATER | | QSP 100 |

As apparent from Table 3, the maximal deposition is obtained by using the combination of capsules coated with 200'000g/mol chitosan C1 and 1'700'000g/mol uncoated chitosan C2. This is also confirmed in the case a mushroom chitosan C1 is used as coating. Finally, a high molecular weight cationic guar is ineffective in promoting capsule deposition on hair, compared to chitosan C2.

## Claims

1. A consumer product comprising a plurality of microcapsules dispersed in a dispersing medium, the microcapsules comprising a core and a shell around the core, the core comprising at least one functional material, wherein the shells of the microcapsules are coated with chitosan and wherein the dispersing medium comprises additional free chitosan, wherein the chitosan that is coating the shells of the microcapsules and the free chitosan comprised in the dispersing medium have different average molecular weights.

2. The consumer product according to claim 1, wherein the free chitosan comprised in the dispersing medium has a deacetylation grade higher than 60%, more particularly higher than 70%, still more particularly higher than 80%.

3. The consumer product according to one of claims 1 or 2, wherein the free chitosan comprised in the dispersing medium has a molecular weight between 500'000 and 5'000'000g/mol, more particularly between 1'000'000 and 4'000'000g/mol, still more particularly between 1'500'000 and 3' 000' 000 g/mol.

4. The consumer product according to one of claims 1 to 3, wherein the chitosan that is coating the shells of the microcapsules has a deacetylation grade between 60% and 100%, more particularly between 70% and 90%, still more particularly between 75% and 85%.

5. The consumer product according to one of claims 1 to 4, wherein the chitosan that is coating the shells of the microcapsules has a molecular weight between 3'000 and 1'000'000 g/mol, more particularly between 10'000 and 500'000g/mol, still more particularly between 30'000 and 300'000 g/mol.

6. The consumer product according to one of claims 1 to 5, wherein the coated microcapsules have a zeta potential of:
- Between 0 mV and +50 mV, more particularly between +5 mV and +40 mV, still more particularly between +10 mV and +30 mV, at a pH of 4 and an ion concentration of 0.001 mol/L;
- Between-20 mV and +20 mV, more particularly between -10 mV and +10 mV, still more particularly between -5 mV and +5 mV, at a pH 5.4 and ion concentration of 0.001 mol/L;
- Less than -20 mV, more particularly less than -40 mV, still more particularly less than -50 mV, even still more particularly between -50 mV and -60 mV, at a pH of 7 and an ion concentration of 0.001 mol/L;
wherein the zeta-potential is the apparent electrostatic potential generated by electrically charged objects in solution, measured by the phase analysis light scattering method.

7. The consumer product according to one of claims 1 to 6, wherein the coated microcapsules have a volume-average size from 0.5 to 25 micrometers, more particularly from 1 to 20 micrometers, still more particularly from 5 to 15 micrometers.

8. The consumer product according to one of claims 1 to 7, wherein the dispersing medium additionally comprises an anionic surfactant, in particular selected from the group consisting of fatty acid sulphates, laureth sulphates, sarcosines and sarcosinates.

9. The consumer product according to one of claims 1 to 8, wherein the shells of the microcapsules comprise a thermosetting resin selected from the group consisting of an aminoplast resin, an polyurea resin, a polyurethane resin, a polyacrylate resin and mixtures thereof.

10. The consumer product according to one of claims 1 to 9, comprising from 0.01 to 5 wt.-%, more particularly from 0.1 to 2.5 wt.-%, still more particularly from 0.2 to 1 wt.-% microcapsules, referred to the total weight of the consumer product.

11. The consumer product according to one of claims 1 to 10, comprising from 0.00001 to 0.005 wt.-%, more particularly from 0.00005 to 0.001 wt.-%, still more particularly from 0.00008 to 0.0005 wt.-% free chitosan, referred to the total weight of the consumer product.

12. A method for making a consumer product, in particular a consumer product according to one of claims 1 to 11, comprising the steps of:
- Providing a plurality of microcapsules, the microcapsules comprising a core and a shell around the core, the core comprising at least one functional material;
- Providing a dispersing medium;
- Dispersing the microcapsules in the dispersing medium;
- Coating the plurality of core-shell microcapsules with chitosan to obtain a plurality of coated core-shell microcapsules;
- Adding additional free chitosan to the dispersing medium, wherein the chitosan that is coating the shells of the microcapsules and the free chitosan comprised in the dispersing medium have different average molecular weights.

13. Use of chitosan for enhancing the deposition and rinse resistance of a plurality of microcapsules on a surface, in particular on a keratinous surface, the microcapsules comprising a core and a shell around the core, the core comprising at least one functional material, the microcapsules being dispersed in a dispersing medium, contained in a consumer product, in a method according to claim 12, by coating the plurality of microcapsules with chitosan to obtain a plurality of coated microcapsules and adding additional free chitosan to the dispersing medium, wherein the chitosan that is coating the shells of the microcapsules and the free chitosan comprised in the dispersing medium have different average molecular weights.

## Patentansprüche

1. Verbraucherprodukt umfassend eine Vielzahl von Mikrokapseln, die in einem Dispergiermedium dispergiert sind, wobei die Mikrokapseln einen Kern und eine Schale um den Kern umfassen, wobei der Kern wenigstens ein funktionelles Material umfasst, wobei die Schalen der Mikrokapseln mit Chitosan beschichtet sind und wobei das Dispergiermedium zusätzliches freies Chitosan umfasst, wobei das Chitosan, das die Schalen der Mikrokapseln beschichtet, und das in dem Dispergiermedium enthaltene freie Chitosan unterschiedliche mittlere Molekulargewichte aufweisen.

2. Verbraucherprodukt gemäß Anspruch 1, wobei das in dem Dispergiermedium enthaltene freie Chitosan einen Deacetylierungsgrad von höher als 60 %, insbesondere höher als 70 %, speziell höher als 80 %, aufweist.

3. Verbraucherprodukt gemäß einem der Ansprüche 1 oder 2, wobei das in dem Dispergiermedium enthaltene freie Chitosan ein Molekulargewicht zwischen 500.000 und 5.000.000 g/mol, insbesondere zwischen 1.000.000 und 4.000.000 g/mol, speziell zwischen 1.500.000 und 3.000.000 g/mol, aufweist.

4. Verbraucherprodukt gemäß einem der Ansprüche 1 bis 3, wobei das Chitosan, das die Schalen der Mikrokapseln beschichtet, einen Deacetylierungsgrad zwischen 60 % und 100 %, insbesondere zwischen 70 % und 90 %, speziell zwischen 75 % und 85 %, aufweist.

5. Verbraucherprodukt gemäß einem der Ansprüche 1 bis 4, wobei das Chitosan, das die Schalen der Mikrokapseln beschichtet, ein Molekulargewicht zwischen 3.000 und 1.000.000 g/mol, insbesondere zwischen 10.000 und 500.000 g/mol, speziell zwischen 30.000 und 300.000 g/mol, aufweist.

6. Verbraucherprodukt gemäß einem der Ansprüche 1 bis 5, wobei die beschichteten Mikrokapseln ein Zeta-Potential aufweisen von:
- zwischen 0 mV und +50 mV, insbesondere zwischen +5 mV und +40 mV, speziell zwischen +10 mV und +30 mV, bei einem pH-Wert von 4 und einer Ionenkonzentration von 0,001 mol/l;
- zwischen -20 mV und +20 mV, insbesondere zwischen -10 mV und +10 mV, speziell zwischen -5 mV und +5 mV, bei einem pH-Wert von 5,4 und einer Ionenkonzentration von 0,001 mol/l;
- weniger als -20 mV, insbesondere weniger als -40 mV, speziell weniger als -50 mV, spezieller zwischen -50 mV und -60 mV, bei einem pH-Wert von 7 und einer Ionenkonzentration von 0,001 mol/l;
wobei das Zeta-Potential das von elektrisch geladenen Gegenständen in Lösung erzeugte scheinbare elektrostatische Potential ist, gemessen durch das Phasenanalyse-Lichtstreuverfahren.

7. Verbraucherprodukt gemäß einem der Ansprüche 1 bis 6, wobei die beschichteten Mikrokapseln eine volumengemittelte Größe von 0,5 bis 25 Mikrometer, insbesondere von 1 bis 20 Mikrometer, spezieller von 5 bis 15 Mikrometer, aufweisen.

8. Verbraucherprodukt gemäß einem der Ansprüche 1 bis 7, wobei das Dispergiermedium zusätzlich ein anionisches Tensid umfasst, insbesondere ausgewählt aus der Gruppe bestehend aus Fettsäuresulfaten, Laurethsulfaten, Sarkosinen und Sarkosinaten.

9. Verbraucherprodukt gemäß einem der Ansprüche 1 bis 8, wobei die Schalen der Mikrokapseln ein wärmehärtendes Harz ausgewählt aus der Gruppe bestehend aus einem Aminoplastharz, einem Polyharnstoffharz, einem Polyurethanharz, einem Polyacrylatharz und Gemischen davon umfasst.

10. Verbraucherprodukt gemäß einem der Ansprüche 1 bis 9, umfassend von 0,01 bis 5 Gew.-%, insbesondere von 0,1 bis 2,5 Gew.-%, spezieller von 0,2 bis 1 Gew.-%, Mikrokapseln, bezogen auf das Gesamtgewicht des Verbraucherprodukts.

11. Verbraucherprodukt gemäß einem der Ansprüche 1 bis 10, umfassend von 0,00001 bis 0,005 Gew.-%, insbesondere von 0,00005 bis 0,001 Gew.-%, spezieller von 0,00008 bis 0,0005 Gew.-%, an freiem Chitosan, bezogen auf das Gesamtgewicht des Verbraucherprodukts.

12. Verfahren zur Herstellung eines Verbraucherprodukts, insbesondere eines Verbraucherprodukts gemäß einem der Ansprüche 1 bis 11, umfassend die Schritte:
- Bereitstellen einer Vielzahl von Mikrokapseln, wobei die Mikrokapseln einen Kern und eine Schale um den Kern umfassen, wobei der Kern wenigstens ein funktionelles Material umfasst;
- Bereitstellen eines Dispergiermediums;
- Dispergieren der Mikrokapseln in dem Dispergiermedium;
- Beschichten der Vielzahl von Kern-Schale-Mikrokapseln mit Chitosan, um eine Vielzahl von beschichteten Kern-Schale-Mikrokapseln zu erhalten;
- Zugeben von zusätzlichem freiem Chitosan zu dem Dispergiermedium, wobei das Chitosan, das die Schalen der Mikrokapseln beschichtet, und das in dem Dispergiermedium enthaltene freie Chitosan unterschiedliche mittlere Molekulargewichte aufweisen.

13. Verwendung von Chitosan zum Verbessern der Abscheidung und der Spülbeständigkeit einer Vielzahl von Mikrokapseln auf einer Oberfläche, insbesondere einer keratinösen Oberfläche, wobei die Mikrokapseln einen Kern und eine Schale um den Kern umfassen, wobei der Kern wenigstens ein funktionelles Material umfasst, wobei die Mikrokapseln in einem Dispergiermedium dispergiert sind, das in einem Verbraucherprodukt enthalten ist, bei einem Verfahren gemäß Anspruch 12, durch Beschichten der Vielzahl von Mikrokapseln mit Chitosan, um eine Vielzahl von beschichteten Mikrokapseln zu erhalten, und Zugeben von zusätzlichem freiem Chitosan zu dem Dispergiermedium, wobei das Chitosan, das die Schalen der Mikrokapseln beschichtet, und das in dem Dispergiermedium enthaltene freie Chitosan unterschiedliche mittlere Molekulargewichte aufweisen.

## Revendications

1. Produit de consommation comprenant une pluralité de microcapsules dispersées dans un milieu de dispersion, les microcapsules comprenant un noyau et une enveloppe autour du noyau, le noyau comprenant au moins une matière fonctionnelle, les enveloppes des microcapsules étant revêtues par du chitosane et le milieu de dispersion comprenant du chitosane libre supplémentaire, le chitosane qui revêt les enveloppes des microcapsules et le chitosane libre compris dans le milieu de dispersion ayant des poids moléculaires moyens différents.

2. Produit de consommation selon la revendication 1, le chitosane libre compris dans le milieu de dispersion ayant un degré de désacétylation supérieur à 60 %, plus particulièrement supérieur à 70 %, encore plus particulièrement supérieur à 80 %.

3. Produit de consommation selon l'une des revendications 1 ou 2, le chitosane libre compris dans le milieu de dispersion ayant un poids moléculaire compris entre 500 000 et 5 000 000 g/mole, plus particulièrement entre 1 000 000 et 4 000 000 g/mole, encore plus particulièrement entre 1 500 000 et 3 000 000 g/mole.

4. Produit de consommation selon l'une des revendications 1 à 3, le chitosane qui revêt les enveloppes des microcapsules ayant un degré de désacétylation compris entre 60 % et 100 %, plus particulièrement entre 70 % et 90 %, encore plus particulièrement entre 75 % et 85 %.

5. Produit de consommation selon l'une des revendications 1 à 4, le chitosane qui revêt les enveloppes des microcapsules ayant un poids moléculaire compris entre 3 000 et 1 000 000 g/mole, plus particulièrement entre 10 000 et 500 000 g/mole, encore plus particulièrement entre 30 000 et 300 000 g/mole.

6. Produit de consommation selon l'une des revendications 1 à 5, les microcapsules revêtues ayant un potentiel zêta :
- entre 0 mV et +50 mV, plus particulièrement entre +5 mV et +40 mV, encore plus particulièrement entre +10 mV et +30 mV, à un pH de 4 et une concentration en ions de 0,001 mole/L ;
- entre -20 mV et +20 mV, plus particulièrement entre -10 mV et +10 mV, encore plus particulièrement entre -5 mV et +5 mV, à un pH 5,4 et une concentration en ions de 0,001 mole/L ;
- inférieur à -20 mV, plus particulièrement inférieur à -40 mV, encore plus particulièrement inférieur à -50 mV, encore plus particulièrement entre -50 mV et -60 mV, à un pH de 7 et une concentration en ions de 0,001 mole/L ;
le potentiel zêta étant le potentiel électrostatique apparent généré par des objets chargés électriquement en solution, mesuré par le procédé de diffusion de lumière à analyse de phase.

7. Produit de consommation selon l'une des revendications 1 à 6, les microcapsules revêtues ayant une taille moyenne en volume de 0,5 à 25 micromètres, plus particulièrement de 1 à 20 micromètres, encore plus particulièrement de 5 à 15 micromètres.

8. Produit de consommation selon l'une des revendications 1 à 7, le milieu de dispersion comprenant de plus un tensioactif anionique, en particulier choisi dans le groupe constitué par des sulfates d'acides gras, des laureth sulfates, des sarcosines et des sarcosinates.

9. Produit de consommation selon l'une des revendications 1 à 8, les enveloppes des microcapsules comprenant une résine thermodurcissable choisie dans le groupe constitué par une résine d'aminoplaste, une résine de polyurée, une résine de polyuréthane, une résine de polyacrylates et des mélanges correspondants.

10. Produit de consommation selon l'une des revendications 1 à 9, comprenant de 0,01 à 5 % en poids, plus particulièrement de 0,1 à 2,5 % en poids, encore plus particulièrement de 0,2 à 1 % en poids de microcapsules, par rapport au poids total du produit de consommation.

11. Produit de consommation selon l'une des revendications 1 à 10, comprenant de 0,00001 à 0,005 % en poids, plus particulièrement de 0,00005 à 0,001 % en poids, encore plus particulièrement de 0,00008 à 0,0005 % en poids de chitosane libre, par rapport au poids total du produit de consommation.

12. Procédé pour la préparation d'un produit de consommation, en particulier d'un produit de consommation selon l'une des revendications 1 à 11, comprenant les étapes de :
- fourniture d'une pluralité de microcapsules, les microcapsules comprenant un noyau et une enveloppe autour du noyau, le noyau comprenant au moins une matière fonctionnelle ;
- fourniture d'un milieu de dispersion ;
- dispersion des microcapsules dans le milieu de dispersion ;
- revêtement de la pluralité de microcapsules noyau-enveloppe par du chitosane pour obtenir une pluralité de microcapsules noyau-enveloppe revêtues ;
- ajout de chitosane libre supplémentaire au milieu de dispersion, le chitosane qui revêt les enveloppes des microcapsules et le chitosane libre compris dans le milieu de dispersion ayant des poids moléculaires moyens différents.

13. Utilisation de chitosane pour améliorer le dépôt et la résistance au rinçage d'une pluralité de microcapsules sur une surface, en particulier sur une surface kératinique, les microcapsules comprenant un noyau et une enveloppe autour du noyau, le noyau comprenant au moins une matière fonctionnelle, les microcapsules étant dispersées dans un milieu de dispersion, contenu dans un produit de consommation, dans un procédé selon la revendication 12, par revêtement de la pluralité de microcapsules par du chitosane pour obtenir une pluralité de microcapsules revêtues et ajout de chitosane libre supplémentaire au milieu de dispersion, le chitosane qui revêt les enveloppes des microcapsules et le chitosane libre compris dans le milieu de dispersion ayant des poids moléculaires moyens différents.
